# FASCICULE DE BREVET EUROPEEN

(11) **EP 1 006 997 B1**
(45) Date de publication et mention de la délivrance du brevet: **02.07.2003**
(21) Numéro de dépôt: 98903067.1
(22) Date de dépôt: 15.01.1998
(51) Int. Cl.: A61K 9/00

(54) **MICROSPHERES EFFERVESCENTES ET LEUR PROCEDE DE FABRICATION**
BRAUSENDE MIKROKUGELN UND VERFAHREN ZU IHRER HERSTELLUNG
EFFERVESCENT MICROSPHERES AND METHOD FOR MAKING THEM

(30) Priorité: 16.01.1997 FR 9700394
(43) Date de publication de la demande: 14.06.2000
(73) Titulaire: PIERRE FABRE MEDICAMENT, 92100 Boulogne-Billancourt (FR)
(72) Inventeur: AIACHE, Jean-Marc, F-63000 Clermont-Ferrand (FR); GAUTHIER, Pascale, F-63000 Clermont-Ferrand (FR); BOUGARET, Jo[l, F-31570 Lanta (FR)
(74) Mandataire: Ahner, Francis
(86) Numéro de dépôt international: FR9800070
(87) Numéro de publication internationale: WO98031342

(56) Documents cités:
- EP-A- 0 415 326
- EP-A- 0 670 160

## Description

La présente invention a pour objet des microsphères effervescentes multicouches et un procédé de préparation de telles microsphères.

Par microsphère, on entendra des microgranules formés d'un matériau support constituant une matrice où sont dispersés le ou les principes actifs éventuellement additionnés de substances auxiliaires. Conformément à la monographie des sphères de la Pharmacopée Européenne, les microsphères ont un diamètre médian inférieur à 1,0 mm et supérieur ou égal à 1,0 µm. Elles sont généralement destinées à la voie orale ou parentérale et sont utilisées soit en qualité de constituants d'une forme pharmaceutique, telle que les comprimés, soit telles quelles associées ou non à d'autres excipients, réparties ou non en doses unitaires, comme les sachets, les gélules ou la poudre pour préparation injectable.

Les formes effervescentes à usage pharmaceutique décrites dans l'art antérieur comprennent exclusivement les granulés et les comprimés obtenus par la compression de ces granulés.

Ces formes effervescentes sont destinées à être dispersées dans l'eau avant absorption. Leur délitement est assuré par un dégagement de dioxyde de carbone résultant de l'action d'un acide - en général un acide organique, le plus utilisé étant l'acide citrique - sur une base - en général un carbonate comme le bicarbonate de sodium, le bicarbonate de potassium ou de lithium, le carbonate de calcium ou de magnésium ou encore le carbonate de lysine pour éviter l'apport de sodium.

Les formes effervescentes connues comprennent également des adjuvants diluants (en général des sucres), des liants, des édulcorants et des aromatisants.

La plupart des procédés classiques de préparation de formes effervescentes comprennent une étape de granulation de poudre, soit par voie humide, soit par voie sèche.

La granulation par voie humide, malgré les difficultés qu'elle présente, est la méthode la plus employée.

Selon une première variante décrite dans "Journal of Pharmaceutical Sciences, 1964, 53, 1524-25", les substances acide et basique, le ou les principes actifs et les adjuvants sont mélangés dans un lit d'air fluidisé. La granulation est amorcée en pulvérisant la poudre avec de l'eau distillée ou une solution aqueuse de diphosphate. L'eau ainsi pulvérisée amorce une réaction d'effervescence qui permet la création de liaisons entre les particules de poudre. L'eau est pulvérisée jusqu'à ce que les granules atteignent la taille souhaitée. L'inconvénient de cette méthode est le manque de reproductibilité des résultats dû à un mauvais contrôle de la réaction d'effervescence.

La demande de brevet EP 673 644 propose de contrôler la réaction d'effervescence du procédé décrit dans la référence précédente, en maintenant le taux d'humidité de l'air d'alimentation du lit d'air fluidisé entre 0 et 1 g/m³, d'une part, et en évaporant l'eau nébulisée à la surface des granulés en formation tout en continuant la pulvérisation d'eau, d'autre part. Les granulés sont séchés une fois la taille souhaitée atteinte. Cette demande décrit l'utilisation de l'eau ou d'un mélange hydroalcoolique comme liquide de mouillage.

Selon une deuxième variante décrite dans le brevet EP 369 228, les substances acide et basique sont granulées séparément puis mélangées après séchage. La mise en oeuvre de ce procédé est coûteuse.

Dans la demande de brevet WO 96/19982 décrivant un procédé de préparation de compositions effervescentes contenant de l'ibuprofen, l'étape de granulation ne concerne que la substance alcaline. Selon ce procédé, la substance alcaline granulée est mélangée à l'ibuprofen pulvérulent.

La granulation par voie sèche comporte deux phases : la compression de la poudre et le broyage-tamisage des comprimés de poudre. Ce procédé présente peu d'intérêt pour la préparation de formes effervescentes du fait de la nature chimique des produits induisant des phénomènes de collage.

Le brevet FR 2 552 308 décrit un procédé de préparation d'un mélange effervescent par une méthode ne faisant pas intervenir d'étape de granulation.

Selon le procédé décrit, le mélange effervescent contient au moins un acide organique solide cristallisé et au moins un carbonate libérant du CO₂ dans la réaction avec l'acide organique et se caractérise en ce que les cristaux de l'acide portent un revêtement contenant du carbonate de calcium et qui adhère à la surface des cristaux d'acide grâce à la couche de liaison formée par réaction partielle du carbonate de calcium du revêtement avec une couche superficielle de chaque cristal d'acide. On prépare le mélange en chauffant l'acide organique dans de l'éthanol et de l'eau à 60°C environ dans un mélangeur sous une pression d'environ 0,1 bar ou inférieure et en introduisant le carbonate de calcium qu'on laisse réagir jusqu'à ce que la pression soit remontée jusqu'à 0,9 bar environ.

La présente invention concerne des microsphères effervescentes multicouches renfermant une substance acide, une substance basique et un agent isolant hydrosoluble dont la dissolution dans l'eau conduit, après effervescence quasi instantanée, à une solution ou une dispersion homogène de principe actif.

Selon une première variante, l'agent isolant hydrosoluble est dispersé dans la totalité de la masse de chaque microsphère, celle-ci présentant une structure bicouche : une couche de substance acide dans laquelle est dispersé l'agent isolant hydrosoluble et une couche de substance alcaline dans laquelle est dispersé l'agent isolant hydrosoluble.

Selon une deuxième variante, l'agent isolant hydrosoluble est sous la forme d'un film mince séparant les substances acide et alcaline. Chaque microsphère présente alors une structure tricouche : une couche de substance acide et une couche de substance alcaline séparée par une couche d'agent isolant hydrosoluble.

Que les microsphères présentent une structure bi- ou tricouche, l'agent isolant hydrosoluble remplit deux fonctions, celle de liant et celle de barrière isolante destinée à éviter une réaction d'effervescence entre la substance alcaline et la substance acide au cours du procédé de préparation mais aussi lors de la conservation des microsphères quelles que soient les conditions de stockage.

L'agent isolant hydrosoluble est choisi parmi la polyvinylpyrrolidone, l'hydroxypropyl cellulose, la méthyl cellulose, le lactose et le saccharose.

La présente invention concerne également un procédé de préparation des microsphères effervescentes précédemment décrites utilisant la méthode de rotogranulation en lit d'air fluidisé.

L'intérêt de la rotogranulation appliquée à ces compositions effervescentes est l'enchaînement continu des opérations dans une seule et même enceinte qui, du fait des composants employés et de certaines précautions, n'induit aucune effervescence. De plus, cette technique de rotogranulation permet de modifier les proportions relatives des différents composés, en particulier les proportions molaires relatives des fractions acide et basique.

Le procédé selon l'invention permet en effet d'obtenir avantageusement des formes effervescentes dont la proportion relative des fractions alcaline et acide est inférieure à la proportion stoechiométrique pratiquée dans l'art antérieur pour des comprimés effervescents fabriqués par la méthode de granulation, sans que la qualité de l'effervescence ne soit altérée.

En particulier, la proportion relative des fractions alcaline et acide pratiquée dans le cadre du procédé selon l'invention est inférieure à 0,6, en particulier inférieur à 0,25.

Toutes les étapes du procédé selon l'invention sont conduites sous pression atmosphérique, sans système de déshydratation particulier, ni précautions spécifiques.

L'appareil utilisé pour la mise en oeuvre du procédé de préparation des microsphères effervescentes est, par exemple, un appareil construit par la société Glatt sur lequel on adapte une cuve rotor.

Un tel appareil est décrit dans le brevet EP 0 505 319 .

La présente invention a tout d'abord pour objet un procédé de préparation de microsphères effervescentes présentant une structure bicouche selon la première variante décrite ci-dessus.

Ledit procédé est réalisé par rotogranulation en lit d'air fluidisé associée à un système de pulvérisation de poudre et un système de pulvérisation tangentielle de liquide de mouillage. Le procédé comprend deux étapes continues, une première étape de sphéronisation de microsphères à partir d'une poudre A et une deuxième étape de sphéronisation d'une poudre B sur les microsphères de poudre A, l'une des poudres A et B étant acide et l'autre alcaline et chacune d'elles pouvant contenir ou être constituée d'un ou plusieurs principes actifs.

Lors de la première sphéronisation, la poudre A est placée dans la cuve de rotogranulation en mouvement et mise en suspension dans le lit d'air. Les composants de la poudre A sont mélangés pendant cinq minutes et la température d'entrée d'air est stabilisée à une température Tₒ.

La poudre A ainsi malaxée est pulvérisée par un liquide de mouillage contenant l'agent isolant hydrosoluble. Les microsphères de poudre A obtenues sont séchées en portant la température d'entrée d'air à Ts puis éventuellement tamisées avec un tamis 1000 µm. Lors de la deuxième sphéronisation, la température d'entrée d'air est ramenée à To. La poudre B et le liquide de mouillage contenant l'agent isolant hydrosoluble sont ensuite simultanément pulvérisés sur les microsphères de poudre A séchées précédemment obtenues. La poudre B est pulvérisée par le biais du système de pulvérisation de poudre installé sur l'appareil Glatt. Les microsphères bicouches obtenues sont séchées en portant la température d'entrée d'air à Ts. Après séchage, les microsphères doivent être conditionnées rapidement mais une petite reprise d'humidité ne gêne pas la conservation.

Au cours des deux sphéronisations, le liquide de mouillage contenant l'agent isolant hydrosoluble est le même, par exemple de la polyvinylpyrrolidone (PVP) en solution dans un alcool ou un mélange hydro-alcoolique, en particulier de la PVP dissoute à 4 % en poids dans de l'éthanol à 60 % en volume.

Les microsphères bicouches obtenues selon le procédé de l'invention ont une granulométrie moyenne comprise entre 20 et 500 µm.

La présente invention a également pour objet un procédé de préparation de microsphères effervescentes présentant une structure tricouche selon la deuxième variante décrite ci-dessus.

Ledit procédé est réalisé selon la méthode de rotogranulation en lit d'air fluidisé associée à un système de pulvérisation tangentielle de liquide de mouillage.

Le procédé comprend trois étapes continues, une première étape de sphéronisation de microsphères à partir d'une poudre A, une deuxième étape de sphéronisation d'un agent isolant hydrosoluble sur les microsphères de poudre A puis une troisième étape de sphéronisation d'une poudre B sur les microsphères A protégées par un film d'agent isolant hydrosoluble, l'une des poudres A et B étant acide et l'autre alcaline et chacune d'elles pouvant contenir ou être constituée d'un ou plusieurs principes actifs.

Lors de la première sphéronisation, la poudre A additionnée d'un liant, par exemple la PVP, est placée dans la cuve en mouvement et mise en suspension dans le lit d'air. Les composants de la poudre A sont mélangés pendant cinq minutes et la température d'entrée d'air est stabilisée à To. La poudre A ainsi malaxée est pulvérisée par un liquide de mouillage. Les microsphères de poudre A obtenues sont séchées en portant la température d'entrée d'air à Ts. Lors de la deuxième sphéronisation, la température d'entrée d'air est ramenée à To. L'agent isolant hydrosoluble est ajouté directement dans la cuve et le liquide de mouillage pulvérisé jusqu'à obtention des microsphères de poudre A recouvertes d'un film d'agent isolant hydrosoluble qui sont séchées en portant la température d'entrée d'air à Ts. Après séchage, les microsphères enrobées sont tamisées puis la poudre B est directement ajoutée dans la cuve de rotogranulation lorsque la température d'entrée d'air est stabilisée à To. On obtient les microsphères tricouches en pulvérisant les microsphères précédentes par un liquide de mouillage. Les microsphères tricouches obtenues sont séchées en portant la température d'entrée d'air à Ts. Après séchage, les microsphères doivent être conditionnées rapidement mais une petite reprise d'humidité ne gêne pas la conservation.

Au cours des deux premières étapes, le liquide de mouillage est par exemple une solution hydroalcoolique, en particulier de l'éthanol à 60 % en volume. Au cours de la dernière étape, l'agent isolant hydrosoluble peut être introduit par le biais de la poudre B auquel cas le liquide de mouillage utilisé sera le même que lors des deux premières étapes, ou alors, l'agent isolant est introduit par le biais du liquide de mouillage qui sera une solution alcoolique ou hydroalcoolique contenant l'agent isolant, par exemple de la PVP dissoute à 4 % en poids dans de l'éthanol à 60 % en volume.

Les microsphères tricouches obtenues selon le procédé de l'invention ont une granulométrie moyenne comprise entre 200 et 1000 µm.

Selon le procédé de fabrication de microsphères quelles soient bi- ou tricouches, la poudre de nature alcaline contient un bicarbonate de sodium ou tout autre carbonate employé habituellement dans la préparation de formes effervescentes, comme le carbonate acide de lithium, le carbonate monosodique, le carbonate glycine lithium, le carbonate monopotassique, le carbonate de calcium, le carbonate de magnésium ; un ou plusieurs principes actifs si ces derniers présentent des caractéristiques alcalines ; tandis que la poudre de nature acide contient un acide organique, par exemple l'acide citrique ou un composé employé comme principe actif, par exemple l'acide ascorbique, l'acétylleucine ou/et un ou plusieurs principes actifs si ces derniers présentent des caractéristiques acides.

Les poudres acide et alcaline peuvent en outre contenir un diluant, par exemple le lactose ou le Glucidex; des arômes et des édulcorants, par exemple l'arôme d'orange, l'acide citrique, le saccharinate de sodium ; des excipients divers.

Selon un mode de réalisation de l'invention, la poudre A est de nature alcaline et la poudre B de nature acide.

Selon un autre mode de réalisation de l'invention, la poudre B est de nature alcaline et la poudre A de nature acide.

La pulvérisation du liquide de mouillage est effectuée au moyen d'une buse de 1,2 mm de diamètre, à un débit moyen compris entre 10 et 30 g/min. La température d'entrée d'air du lit fluidisé est comprise entre 55 et 65°C lors des étapes de sphéronisation (To) et comprise entre 75 et 85°C lors des phases de séchage (Ts).

Les microsphères obtenues selon le procédé de l'invention contiennent 5 à 75 % de substance alcaline, 10 à 75 % de substance acide, 3 à 15 % d'agent isolant hydrosoluble, 5 à 50 % de diluant, 1 à 30 % d'arômes et d'édulcorants.

L'humidité relative des microsphères obtenues selon le procédé de l'invention mesurée pendant quinze minutes par la méthode de la balance infrarouge à 90°C est comprise entre 1 et 2 % à la sortie de la cuve de rotogranulation.

Le rendement total du procédé est calculé à partir de la fraction de particules de taille inférieure à 2500 µm, le rendement utile des sphères correspond à la fraction de particules comprise entre 200 et 1000 µm, pour le procédé de préparation de microsphères tricouches, entre 20 et 500 µm pour le procédé de préparation de microsphères bicouches.

La faisabilité du procédé selon l'invention est évaluée selon la facilité d'obtention des microsphères, la vitesse de réalisation d'un lot et le rendement de chaque étape.

L'analyse des lots comporte l'analyse granulométrique d'un échantillon de 100 g de sphères par la méthode des tamis superposés (échantillon provenant de la fraction totale d'un lot), puis une étude morphologique des microsphères obtenues portant sur l'aspect global, la sphéricité, la cohésion et la régularité des particules est effectuée par examen à la loupe binoculaire.

Selon une variante de l'invention, les microsphères effervescentes bi- ou tricouches sont fabriquées par la technique de montage associée à un système de pulvérisation tangentielle de liquide de mouillage. La poudre A et la poudre B peuvent être successivement montées sur de sphères de principe actif enrobées d'agent isolant hydrosoluble, ou sur des neutres.

Les exemples suivants illustrent l'invention sans en limiter la portée.

Les pourcentages sont exprimés en poids.

### Exemple 1 : Microsphères effervescentes bicouches contenant de l'acide ascorbique (vitamine C)

On prépare des microsphères alcalines sur lesquelles on dépose le principe actif acide (vitamine C) .

Le tableau ci-après donne le détail de la formulation utilisée.

| FORMULATION | COMPOSANT | POURCENTAGE |
|---|---|---|
| Poudre A | | |
| Composé alcalin | Bicarbonate de sodium | 20 % |
| Diluant | Lactose | 6 % |
| Edulcorant | Glucidex 6® | 6 % |

| Poudre B | | |
|---|---|---|
| Composé acide Principe actif | Acide ascorbique | 50 % |
| Arôme | Arôme orange | 1 % |
| Edulcorants | Saccharinate de sodium Glucidex 6® | 0,3 % 6,35 % |
| Diluant | Lactose | 6,35 % |

Le liquide de mouillage utilisé au cours des deux rotogranulations successives est une solution hydroalcoolique de PVP contenant 4 % de PVP dans de l'éthanol à 60 % en volume.

Ce mélange est pulvérisé à un débit moyen de 25 grammes par minutes.

Dans cette formulation, le lactose est associé en part égale au Glucidex 6®, il est toutefois possible d'employer le lactose seul.

Les formulations de poudres A et B ont été réalisées sur des lots de taille variable de 1000 à 5000 g avec, selon les cas, utilisation d'un matériel de la société Glatt.

Les sphères effervescentes obtenues présentent un aspect assez régulier et une granulométrie majoritaire des fractions comprises entre 200 et 500 µm. L'humidité relative est de 1,6 % à la sortie de la cuve de rotogranulation.

### Exemple 2 : Microsphères effervescentes bicouches contenant de l'acétylleucine

On prépare des microsphères alcalines sur lesquelles on dépose le principe actif acide (acétylleucine) dans les mêmes conditions que dans l'exemple 1.

Le tableau ci-dessous donne le détail de la formulation utilisée.

| FORMULATION | COMPOSANT | POURCENTAGE |
|---|---|---|
| Poudre A | | |
| Composé alcalin | Bicarbonate de sodium | 20 % |
| Diluant | Lactose | 9,85 % |

| Poudre B | | |
|---|---|---|
| Composé acide Principe actif | Acétylleucine | 50 % |
| Arôme | Arôme orange | 1 % |
| Edulcorant | Saccharinate de sodium | 0,3 % |
| Diluant | Lactose | 9,85 % |

La répartition granulométrique du lot est majoritaire pour les fractions 25 à 500 µm.

L'humidité relative est de 1,9 % à la sortie de la cuve de rotogranulation.

Selon la taille des lots variant de 1000 à 10000 g, un appareil GPCG 1 ou GPCG 5 de la société Glatt avec un montage d'une cuve rotor.

### Exemple 3 : Microsphères effervescentes tricouches contenant de l'acide ascorbique (vitamine C)

On fabrique des microsphères effervescentes tricouches comprenant un coeur alcalin isolé du principe actif acide, l'acide ascorbique, par un film de PVP.

| FORMULATION | COMPOSANT | POURCENTAGE |
|---|---|---|
| Poudre A | | |
| Composé alcalin | Bicarbonate de sodium | 25 % |
| Liant | PVP K30 | 1,316 % |
| Diluant | Lactose | 7,950 % |

| Agent isolant | | |
|---|---|---|
| hydrosoluble | PVP K30 | 6,958 % |

| Poudre B | | |
|---|---|---|
| Composé acide Principe actif | Acide ascorbique | 50 % |
| Arôme | Arôme orange | 1 % |
| Edulcorants | Saccharinate de sodium | 0,2 % |
| | Acide citrique | 1 % |
| Diluant | Lactose | 6,950 % |

L'essai est réalisé dans un appareil de type GPCG1 de la société Glatt avec le montage de la cuve rotor.

On pulvérise au total lors des trois étapes, 1460 g d'éthanol à 60 % en volume, à un débit moyen de 15 grammes par minute.

La taille du lot final est de 1000 g.

Le rendement utile correspondant à la fraction de particules comprise entre 200 et 1000 µm est de 65 %. L'humidité relative est de 1,5 % à la sortie de la cuve.

## Revendications

1. Microsphères effervescentes multicouches renfermant une substance acide, une substance basique et un agent isolant hydrosoluble dont la dissolution dans l'eau conduit, après effervescence quasi instantanée, à une solution ou une dispersion homogène de principe(s) actif(s), **caractérisées en ce que** les substances acide et basique contiennent ou sont constituées de principe(s) actif(s).

2. Microsphères selon la revendication 1, **caractérisées en ce que** l'agent isolant hydrosoluble est dispersé dans la totalité de la masse de chaque microsphère.

3. Microsphères selon la revendication 1, **caractérisées en ce que** l'agent isolant hydrosoluble est sous la forme d'un film mince séparant les substances acide et basique.

4. Microsphères selon les revendications 1 à 3, **caractérisées en ce que** l'agent isolant hydrosoluble est choisi parmi la polyvinylpyrrolidone, l'hydroxypropy cellulose, la méthyl cellulose, le lactose et le saccharose.

5. Procédé de préparation de microsphères selon les revendications 1 à 4, **caractérisé en ce qu'**il utilise la méthode de rotogranulation en lit d'air fluidisé.

6. Procédé de préparation de microsphères selon la revendication 1 ou 2, par la méthode de rotogranulation en lit d'air fluidisé associée à un système de pulvérisation de poudre et un système de pulvérisation tangentielle de liquide de mouillage, **caractérisé en ce qu'**il comprend deux étapes continues, une première étape de sphéronisation de microsphères à partir d'une poudre A et une deuxième étape de sphéronisation d'une poudre B sur les microsphères de poudre A, l'une des poudres A et B étant acide et l'autre alcaline.

7. Procédé selon la revendication 4, **caractérisé en ce que** la poudre A est directement introduite dans la cuve de rotogranulation puis pulvérisée par un liquide de mouillage contenant l'agent isolant hydrosoluble, tandis que la poudre B et un liquide de mouillage contenant l'agent isolant hydrosoluble sont simultanément et respectivement pulvérisés via le système de pulvérisation de poudre et le système de pulvérisation tangentielle de liquide.

8. Procédé selon les revendications 6 et 7, **caractérisé en ce que** les microsphères obtenues ont une granulométrie moyenne comprise entre 20 et 500 µm.

9. Procédé de préparation de microsphères selon la revendication 1 ou 3, par la méthode de rotogranulation en lit d'air fluidisé associée à un système de pulvérisation tangentielle de liquide de mouillage, **caractérisé en ce qu'**il comprend trois étapes continues, une première étape de sphéronisation de microsphères à partir d'une poudre A, une deuxième étape de sphéronisation d'un agent isolant hydrosoluble sur les microsphères de poudre A puis une troisième étape de sphéronisation d'une poudre B sur les microsphères A protégées par un film d'agent isolant hydrosoluble, l'une des poudres A et B étant acide et l'autre alcaline.

10. Procédé selon la revendication 9, **caractérisé en ce que** la poudre A et l'agent isolant hydrosoluble sont pulvérisés par une solution alcoolique ou hydroalcoolique.

11. Procédé selon les revendications 9 et 10, **caractérisé en ce que** la poudre B contient l'agent isolant hydrosoluble et est pulvérisée par une solution alcoolique ou hydroalcoolique.

12. Procédé selon les revendications 9 et 10, **caractérisé en ce que** la poudre B est pulvérisée par un liquide de mouillage contenant l'agent isolant hydrosoluble.

13. Procédé selon les revendications 9 à 12, **caractérisé en ce que** les microsphères obtenues ont une granulométrie moyenne comprise entre 200 et 1000 µm.

14. Procédé selon la revendication 7 ou 12, **caractérisé en ce que** le liquide de mouillage contenant l'agent isolant hydrosoluble est de la polyvinylpyrrolidone en solution dans un alcool ou un mélange hydroalcoolique, en particulier de la polyvinylpyrrolidone dissoute à 4 % en poids dans de l'éthanol à 60 % en volume.

15. Procédé selon la revendication 6 ou 9, **caractérisé en ce que** la poudre de nature alcaline contient un bicarbonate de sodium ou tout autre carbonate employé habituellement dans la préparation de formes effervescentes, comme le carbonate acide de lithium, le carbonate monosodique, le carbonate glycine lithium, le carbonate monopotassique, le carbonate de calcium, le carbonate de magnésium ; un ou plusieurs principes actifs si ces derniers présentent des caractéristiques alcalines.

16. Procédé selon la revendication 6 ou 9, **caractérisé en ce que** la poudre de nature acide contient un acide organique, par exemple l'acide citrique ou un composé employé comme principe actif, par exemple l'acide ascorbique, l'acétylleucine ou/et un ou plusieurs principes actifs si ces derniers présentent des caractéristiques acides.

17. Procédé selon la revendication 15 ou 16, **caractérisé en ce que** les poudres de natures alcaline et acide contiennent en outre un diluant, par exemple le lactose ; des arômes et des édulcorants, par exemple l'arôme d'orange, l'acide citrique, le saccharinate de sodium ; des excipients divers.

18. Procédé selon la revendication 6 ou 9, **caractérisé en ce que** les microsphères obtenues contiennent 5 à 75 % de substance alcaline, 10 à 75 % de substance acide, 3 à 15 % d'agent isolant hydrosoluble, 5 à 50 % de diluant, 1 à 30 % d'arômes et d'édulcorants.

19. Procédé selon la revendication 6 ou 9, **caractérisé en ce que** la poudre A est de nature alcaline et la poudre B de nature acide.

20. Procédé selon la revendication 6 ou 9, **caractérisé en ce que** la poudre A est de nature acide et la poudre B de nature alcaline.

21. Procédé selon la revendication 7, 10, 11 ou 12, **caractérisé en ce que** la pulvérisation par le liquide de mouillage est effectuée au moyen d'une buse de 1,2 mm de diamètre, à un débit moyen compris entre 10 et 30 g/min.

22. Procédé selon la revendication 6 ou 9, **caractérisé en ce que** la température d'entrée d'air du lit fluidisé est comprise entre 55 et 65°C lors des étapes de sphéronisation, et entre 75 et 85°C lors des phases de séchage associées aux étapes de sphéronisation.

23. Procédé selon la revendication 6 ou 9, **caractérisé en ce que** l'humidité relative des microsphères obtenues est comprise entre 1 et 2 % à la sortie de la cuve de rotogranulation.

24. Procédé de préparation de microsphères selon les revendications 1 à 4, **caractérisé en ce qu'**il utilise la technique de montage associée à un système de pulvérisation tangentielle de liquide de mouillage.

25. Procédé selon la revendication 24, **caractérisé en ce que** la poudre A et la poudre B sont successivement montées sur des sphères de principe actif enrobées d'agent isolant hydrosoluble, ou sur des neutres.

## Patentansprüche

1. Aufschäumende, eine Mehrzahl von Schichten aufweisende Mikrokugeln, die eine saure Substanz, eine basische Substanz und ein wasserlösliches isolierendes Mittel umfassen, deren Auflösung in Wasser nach praktisch unverzüglichem Aufschäumen zu einer Lösung oder einer homogenen Dispersion von einem oder mehreren Wirkstoff(en) führt, **dadurch gekennzeichnet, dass** die saure Substanz und die basische Substanz einen oder mehrere Wirkstoff(e) enthalten oder daraus bestehen.

2. Mikrokugeln nach Anspruch 1, **dadurch gekennzeichnet, dass** das wasserlösliche isolierende Mittel in der Gesamtheit der Masse von jeder Mikrokugel dispergiert ist.

3. Mikrokugeln nach Anspruch 1, **dadurch gekennzeichnet, dass** das wasserlösliche isolierende Mittel in Form eines dünnen Films vorliegt, der die saure Substanz und die basische Substanz trennt.

4. Mikrokugeln nach den Ansprüchen 1 bis 3, **dadurch gekennzeichnet, dass** das wasserlösliche isolierende Mittel unter Polyvinylpyrrolidon, Hydroxypropylcellulose, Methylcellulose, Lactose und Saccharose ausgewählt wird.

5. Verfahren zur Herstellung von Mikrokugeln nach den Ansprüchen 1 bis 4, **dadurch gekennzeichnet, dass** es das Rotationsgranulierungsverfahren im luftdurchströmten Wirbelbett einsetzt.

6. Verfahren zur Herstellung von Mikrokugeln nach Anspruch 1 oder 2 durch das Rotationsgranulierungsverfahren im luftdurchströmten Wirbelbett kombiniert mit einem Pulverzerstäubungssystem und einem System tangentialer Zerstäubung von Benetzungsflüssigkeit, **dadurch gekennzeichnet, dass** es zwei kontinuierliche Schritte umfasst, einen ersten Schritt einer Kugelbildung von Mikrokugeln ausgehend von einem Pulver A und einen zweiten Schritt einer Kugelbildung mittels eines Pulvers B auf den Mikrokugeln aus Pulver A, wobei eines der Pulver A und B sauer und das andere alkalisch ist.

7. Verfahren nach Anspruch 4, **dadurch gekennzeichnet, dass** das Pulver A direkt in den Rotationsgranulierungsbehälter eingespeist wird, dann mittels einer Benetzungsflüssigkeit, die das wasserlösliche isolierende Mittel enthält, zerstäubt wird, wohingegen das Pulver B und eine Benetzungsflüssigkeit, die das wasserlösliche isolierende Mittel enthält, gleichzeitig und jeweils einzeln über das Pulverzerstäubungssystem und das tangentiale Flüssigkeitszerstäubungssystem zerstäubt werden.

8. Verfahren nach den Ansprüchen 6 und 7, **dadurch gekennzeichnet, dass** die erhaltenen Mikrokugeln eine mittlere Korngrößenverteilung zwischen 20 und 500 µm haben.

9. Verfahren zur Herstellung von Mikrokugeln nach Anspruch 1 oder 3 durch das Rotationsgranulierungsverfahren im luftdurchströmten Wirbelbett kombiniert mit einem System tangentialer Zerstäubung von Benetzungsflüssigkeit, **dadurch gekennzeichnet, dass** es drei kontinuierliche Schritte umfasst, einen ersten Schritt einer Kugelbildung von Mikrokugeln ausgehend von einem Pulver A, einen zweiten Schritt einer Kugelbildung mittels eines wasserlöslichen isolierenden Mittels auf den Mikrokugeln aus Pulver A, dann einen dritten Schritt einer Kugelbildung mittels eines Pulvers B auf den Mikrokugeln A, die durch einen Film von wasserlöslichem isolierendem Mittel geschützt sind, wobei eines der Pulver A und B sauer und das andere alkalisch ist.

10. Verfahren nach Anspruch 9, **dadurch gekennzeichnet, dass** das Pulver A und das wasserlösliche isolierende Mittel mittels einer alkoholischen oder wässrig-alkoholischen Lösung zerstäubt werden.

11. Verfahren nach den Ansprüchen 9 und 10, **dadurch gekennzeichnet, dass** das Pulver B das wasserlösliche isolierende Mittel enthält und mittels einer alkoholischen oder wässrig-alkoholischen Lösung zerstäubt wird.

12. Verfahren nach den Ansprüchen 9 und 10, **dadurch gekennzeichnet, dass** das Pulver B mittels einer Benetzungsflüssigkeit, die das wasserlösliche isolierende Mittel enthält, zerstäubt wird.

13. Verfahren nach den Ansprüchen 9 bis 12, **dadurch gekennzeichnet, dass** die erhaltenen Mikrokugeln eine mittlere Korngrößenverteilung zwischen 200 und 1000 µm aufweisen.

14. Verfahren nach Anspruch 7 oder 12, **dadurch gekennzeichnet, dass** die Benetzungsflüssigkeit, die das wasserlösliche isolierende Mittel enthält, Polyvinylpyrrolidon in Lösung in einem Alkohol oder einer Wasser-Alkohol-Mischung, insbesondere Polyvinylpyrrolidon gelöst zu 4 Gew.-% in 60%-igem (bezogen auf das Volumen) Ethanol ist.

15. Verfahren nach Anspruch 6 oder 9, **dadurch gekennzeichnet, dass** das Pulver von alkalischer Natur ein Natriumbicarbonat oder ein jegliches anderes Carbonat, das gewöhnlich bei der Herstellung von aufschäumenden Formen eingesetzt wird, wie saures Lithiumcarbonat, Mononatriumcarbonat, Lithiumglycincarbonat, Monokaliumcarbonat, Calciumcarbonat, Magnesiumcarbonat; einen oder mehrere Wirkstoffe, wenn diese Letzteren alkalische Eigenschaften aufweisen, enthält.

16. Verfahren nach Anspruch 6 oder 9, **dadurch gekennzeichnet, dass** das Pulver saurer Natur eine organische Säure, beispielsweise Citronensäure, oder eine Verbindung, die als Wirkstoff eingesetzt wird, beispielsweise Ascorbinsäure, Acetylleucin und/oder einen oder mehrere Wirkstoffe, wenn diese Letzteren saure Eigenschaften aufweisen, enthält.

17. Verfahren nach Anspruch 15 oder 16, **dadurch gekennzeichnet, dass** die Pulver alkalischer und saurer Natur außerdem ein Verdünnungsmittel, beispielsweise Lactose; Aromastoffe und Süßstoffe, beispielsweise Orangenaroma, Citronensäure, Natriumsaccharinat; verschiedene Trägersubstanzen enthalten.

18. Verfahren nach Anspruch 6 oder 9, **dadurch gekennzeichnet, dass** die erhaltenen Mikrokugeln 5 bis 75% alkalische Substanz, 10 bis 75% saure Substanz, 3 bis 15% wasserlösliches isolierendes Mittel, 5 bis 50% Verdünnungsmittel, 1 bis 30% Aromastoffe und Süßstoffe enthalten.

19. Verfahren nach Anspruch 6 oder 9, **dadurch gekennzeichnet, dass** das Pulver A alkalischer Natur und das Pulver B saurer Natur ist.

20. Verfahren nach Anspruch 6 oder 9, **dadurch gekennzeichnet, dass** das Pulver A saurer Natur und das Pulver B alkalischer Natur ist.

21. Verfahren nach Anspruch 7, 10, 11 oder 12, **dadurch gekennzeichnet, dass** das Zerstäuben durch die Benetzungsflüssigkeit mittels einer Düse von 1,2 mm Durchmesser mit einer mittleren Fördermenge zwischen 10 und 30 g/min ausgeführt wird.

22. Verfahren nach Anspruch 6 oder 9, **dadurch gekennzeichnet, dass** die Lufteintrittstemperatur des Wirbelbetts zwischen 55 und 65°C während der Kugelbildungsschritte und zwischen 75 und 85°C während der Trocknungsphasen, die mit den Kugelbildungsschritten kombiniert sind, liegt.

23. Verfahren nach Anspruch 6 oder 9, **dadurch gekennzeichnet dass** die relative Feuchtigkeit der erhaltenen Mikrokugeln beim Austritt aus dem Rotationsgranulierungsbehälter zwischen 1 und 2% beträgt.

24. Verfahren zur Herstellung von Mikrokugeln nach den Ansprüchen 1 bis 4, **dadurch gekennzeichnet, dass** es die Aufbautechnik, die mit einem System tangentialer Zerstäubung von Benetzungsflüssigkeit kombiniert ist, einsetzt.

25. Verfahren nach Anspruch 24, **dadurch gekennzeichnet, dass** das Pulver A und das Pulver B nacheinander auf Kugeln von Wirkstoff, die mit wasserlöslichem isolierendem Mittel umhüllt sind, oder auf neutrale Kerne aufgebracht werden.

## Claims

1. Multilayer effervescent microspheres containing an acidic substance, a basic substance and a water-soluble isolating agent whose dissolution in water leads, after almost immediate effervescence, to a solution or a homogeneous dispersion of active principle(s), **characterized in that** the acidic and basic substances contain or consist of active principle(s).

2. Microspheres according to Claim 1, **characterized in that** the water-soluble isolating agent is dispersed in the entire bulk of each microsphere.

3. Microspheres according to Claim 1, **characterized in that** the water-soluble isolating agent is in the form of a thin film separating the acidic and basic substances.

4. Microspheres according to Claims 1 to 3, **characterized in that** the water-soluble isolating agent is chosen from polyvinylpyrrolidone, hydroxypropyl cellulose, methyl cellulose, lactose and sucrose.

5. Process for preparing microspheres according to Claims 1 to 4, **characterized in that** it uses the method of rotary granulation in a fluidized air bed.

6. Process for preparing microspheres according to Claim 1 or 2, by the method of rotary granulation in a fluidized air bed combined with a system for spraying powder and a system for the tangential spraying of wetting liquid, **characterized in that** it comprises two continuous steps, a first step of spheronization of microspheres using a powder A and a second step of spheronization of a powder B on the microspheres of powder A, one of the powders A and B being acidic and the other alkaline.

7. Process according to Claim 4, **characterized in that** the powder A is introduced directly into the rotary granulation tank and then sprayed with a wetting liquid containing the water-soluble isolating agent, while the powder B and a wetting liquid containing the water-soluble isolating agent are simultaneously and respectively sprayed via the system for spraying powder and the system for the tangential spraying of liquid.

8. Process according to Claims 6 and 7, **characterized in that** the microspheres obtained have an average particle size of between 20 and 500 µm.

9. Process for preparing microspheres according to Claim 1 or 3, by the method of rotary granulation in a fluidized air bed combined with a system for the tangential spraying of wetting liquid, **characterized in that** it comprises three continuous steps, a first step of spheronization of microspheres using a powder A, a second step of spheronization of a water-soluble isolating agent on the microspheres of powder A, and then a third step of spheronization of a powder B on the microspheres A protected with a film of water-soluble isolating agent, one of the powders A and B being acidic and the other alkaline.

10. Process according to Claim 9, **characterized in that** the powder A and the water-soluble isolating agent are sprayed with an alcoholic or aqueous-alcoholic solution.

11. Process according to Claims 9 and 10, **characterized in that** the powder B contains the water-soluble isolating agent and is sprayed with an alcoholic or aqueous-alcoholic solution.

12. Process according to Claims 9 and 10, **characterized in that** the powder B is sprayed with a wetting liquid containing the water-soluble isolating agent.

13. Process according to Claims 9 to 12, **characterized in that** the microspheres obtained have an average particle size of between 200 and 1000 µm.

14. Process according to Claim 7 or 12, **characterized in that** the wetting liquid containing the water-soluble isolating agent is polyvinylpyrrolidone dissolved in an alcohol or an aqueous-alcoholic mixture, in particular polyvinylpyrrolidone dissolved to 4% by weight in ethanol at 60% by volume.

15. Process according to Claim 6 or 9, **characterized in that** the powder of alkaline nature contains a sodium bicarbonate or any other carbonate usually used in the preparation of effervescent forms, such as lithium hydrogen carbonate, monosodium carbonate, lithium glycine carbonate, monopotassium carbonate, calcium carbonate, magnesium carbonate; one or more active principles if the latter have alkaline properties.

16. Process according to Claim 6 or 9, **characterized in that** the powder of acidic nature contains an organic acid, for example citric acid or a compound used as active principle, for example ascorbic acid, acetylleucine and/or one or more active principles if the latter have acidic properties.

17. Process according to Claim 15 or 16, **characterized in that** the powders of alkaline and acidic nature also contain a diluent, for- example lactose; flavourings and sweeteners, for example orange flavouring, citric acid, sodium saccharinate; various excipients.

18. Process according to Claim 6 or 9, **characterized in that** the microspheres obtained contain 5 to 75% of alkaline substance, 10 to 75% of acidic substance, 3 to 15% of water-soluble isolating agent, 5 to 50% of diluent and 1 to 30% of flavourings and sweeteners.

19. Process according to Claim 6 or 9, **characterized in that** the powder A is of alkaline nature and the powder B of acidic nature.

20. Process according to Claim 6 or 9, **characterized in that** the powder A is of acidic nature and the powder B of alkaline nature.

21. Process according to Claim 7, 10, 11 or 12, **characterized in that** the wetting liquid is sprayed by means of a nozzle 1.2 mm in diameter, at an average flow rate of between 10 and 30 g/min.

22. Process according to Claim 6 or 9, **characterized in that** the air inlet temperature of the fluidized bed is between 55 and 65°C during the spheronization steps, and between 75 and 85°C during the drying phases associated with the spheronization steps.

23. Process according to Claim 6 or 9, **characterized in that** the relative humidity of the microspheres obtained is between 1 and 2% at the rotary granulation tank outlet.

24. Process for preparing microspheres according to Claims 1 to 4, **characterized in that** it uses the mounting technique combined with a system for the tangential spraying of wetting liquid.

25. Process according to Claim 24, **characterized in that** the powder A and the powder B are mounted successively on spheres of active principle coated with water-soluble isolating agent, or on neutral spheres.
